# EUROPEAN PATENT APPLICATION

(11) **EP 2 502 645 A1**
(43) Date of publication of application: **26.09.2012**
(21) Application number: 12154710.3
(22) Date of filing: 09.02.2012
(51) Int. Cl.: A61M 25/09

(54) **Guidewire**

(30) Priority: 23.03.2011 JP 2011065048
(71) Applicant: Asahi Intecc Co., Ltd., Nagoya-shi, Aichi 463-0024 (JP)
(72) Inventor: Matsumoto, Satoru, Nagoya-shi, Aichi 463-0024 (JP)
(74) Representative: Winter, Brandl, Fürniss, Hübner, Röss, Kaiser, Polte - Partnerschaft

(57) **Abstract**

A guidewire (1) includes a core shaft (10) and a coil (20). The core shaft (10) has a diameter that decreases from a proximal end portion (11) toward a distal end portion (12), and the coil (20) is wound around an outer periphery of the distal end portion (12). The distal end portion (12) includes a most distal end portion (13) that is positioned at a most distal end of the distal end portion (12), and an intermediate portion (14) that is connected to the most distal end portion (13). The cross-sectional shape of the most distal end portion (13) is a rectangular shape. The cross-sectional shape of the intermediate portion (14) gradually changes from the rectangular shape to a circular shape in a direction from the most distal end portion (13) toward the proximal end portion (11). The length (X1) of the most distal end portion (13) is shorter than the length (X2) of the intermediate portion (14).

## Description

### BACKGROUND OF THE INVENTION

### 1. Field of the Invention

The present invention relates to a guidewire.

### 2. Description of the Related Art

A guidewire is a known example of a medical device that is used for percutaneous transluminal coronary angioplasty (hereinafter referred to as PTCA). A guidewire is used to guide a device such as a balloon, a stent, or the like to a lesion.

For example, JP 2006-289115 A describes such a guidewire. The guidewire includes a core shaft and a synthetic resin coating. The core shaft includes a proximal end portion and a distal end portion, and the synthetic resin coating covers the outer periphery of the core shaft.

The distal end portion of the core shaft includes a most distal end portion, an intermediate portion, and a cylindrical portion. The most distal end portion has a flat plate-like shape. The intermediate portion is connected to the most distal end portion and has a shape that gradually changes from a flat plate-like shape to a cylindrical shape. The cylindrical portion is connected to the intermediate portion. The most distal end portion has a length that is sufficiently longer than that of the intermediate portion. In general, the term "distal portion" refers to a part of a guidewire near the distal end of the guidewire and the term "proximal portion" refers to a part of the guidewire near the proximal end of the guidewire. The distal portion of the guidewire is inserted into a human body, and the proximal portion of the guidewire is operated by an operator such as a doctor.

A guidewire configured as described above has a flexible distal end portion, so that the guidewire can be easily inserted into a human body.

When the existing guidewire described in JP 2006-289115 A is inserted into a complexly-curved blood vessel and a proximal portion of the guidewire is rotated, the number of rotations of the proximal end portion does not coincide with the number of rotations of the distal end portion and the distal portion is rotated only slightly at the initial stage of rotation. However, if the proximal portion is rotated further, the distal end portion, which has rotated only slightly, may abruptly rotate and the guidewire may spring forward.

The inventor examined the cause of such springing forward of a guidewire and found that springing forward occurs due to the shape of a distal end portion of the guidewire. This will be described below with reference to the drawings.

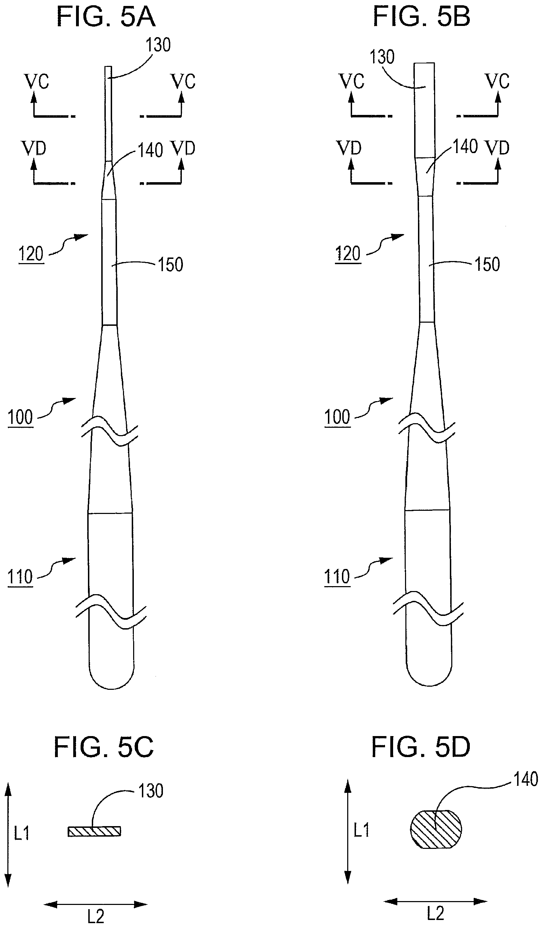

Fig. 5A is a schematic sectional view of a core shaft of an existing guidewire that is cut vertically along the longitudinal direction of the core shaft, and Fig. 5B is a schematic sectional view of the core shaft illustrated Fig. 5A that is cut horizontally along the longitudinal direction. Fig. 5C is a sectional view of a most distal end portion of the core shaft illustrated in Figs. 5A and 5B taken along line VC-VC, and Fig. 5D is a sectional view of an intermediate portion of the core shaft illustrated in Figs. 5A and 5B taken along line VD-VD.

Referring to Figs. 5A and 5B, a core shaft 100 used in the existing guidewire includes a proximal end portion 110 and a distal end portion 120. The diameter of the core shaft 100 decreases from the proximal end portion 110 toward the distal end portion 120.

The distal end portion 120 includes a most distal end portion 130, an intermediate portion 140, and a cylindrical portion 150. The most distal end portion 130 has a flat plate-like shape. The intermediate portion 140 is connected to the most distal end portion 130 and has a shape that gradually changes in a direction from a flat plate-like shape to a cylindrical shape. The cylindrical portion 150 is connected to the intermediate portion 140.

Referring to Fig. 5C, the most distal end portion 130 has a flexibility that is directionally dependent. To be specific, the most distal end portion 130 is easily bent when an external force is applied in directions (vertical directions L1 in Fig. 5C) perpendicular to a principal surface, which includes long sides and has a larger area. In contrast, the most distal end portion 130 is not easily bent when an external force is applied in directions (horizontal directions L2 in Fig. 5C) perpendicular to a side surface, which includes short sides and has a smaller area. In addition, because the most distal end portion 130 has a length that is sufficiently longer than that of the intermediate portion 140, the most distal end portion 130 has a high flexibility. On the other hand, when the proximal end portion 110 is rotated in either direction around the longitudinal axis of the core shaft 100, the most distal end portion 130, which is flexible, is likely to be twisted by a twisting force that has been generated. Referring to Fig. 5D, the directionality of the flexibility of the intermediate portion 140 is low, because the shape of the intermediate portion 140 is close to a cylindrical shape. Therefore, the intermediate portion 140 has a flexibility that is lower than that of the most distal end portion 130, and the intermediate portion 140 is less likely to be twisted.

Because the guidewire includes the core shaft 100 configured as described above, the flexibility of a distal portion of the guidewire is directionally dependent. Therefore, when the proximal portion is rotated, the distal portion is likely to be twisted and is only slightly rotated at the initial stage of rotation. However, if the proximal portion is rotated further, the distal end portion, which has rotated only slightly, may abruptly rotate and the guidewire may spring forward.

Thus, the existing guidewire is likely to damage an inner wall of a blood vessel due to springing forward. If the guidewire springs forward substantially, the guidewire may make a hole in the inner wall of the blood vessel.

### SUMMARY OF THE INVENTION

Starting out from JP 2006-289115 A the present invention has the object to provide a guidewire in which the risk of damaging an inner wall of a blood vessel and making a hole in the inner wall is reduced. This object is solved by a guidewire according to claim 1. Preferred embodiments thereof are subject-matter of dependent claims.

The inventor carried out an intensive study to solve the problem described above, and found that springing forward of a guidewire can be prevented by making the length of the flexible most distal end portion shorter than that of the intermediate portion, and has made a guidewire according to the present invention.

According to the present invention, a guidewire includes a core shaft and a coil. The core shaft has a diameter that decreases from a proximal end portion toward a distal end portion, and the coil is wound around an outer periphery of the distal end portion. The distal end portion includes a most distal end portion that is positioned at a most distal end of the distal end portion, and an intermediate portion that is connected to the most distal end portion. The cross-sectional shape of the most distal end portion is a rectangular shape. The cross-sectional shape of the intermediate portion gradually changes from the rectangular shape to a circular shape in a direction from the most distal end portion toward the proximal end portion. The length of the most distal end portion is shorter than the length of the intermediate portion.

The structure and advantages of the guidewire according to the present invention will be described below in detail with reference to the drawings.

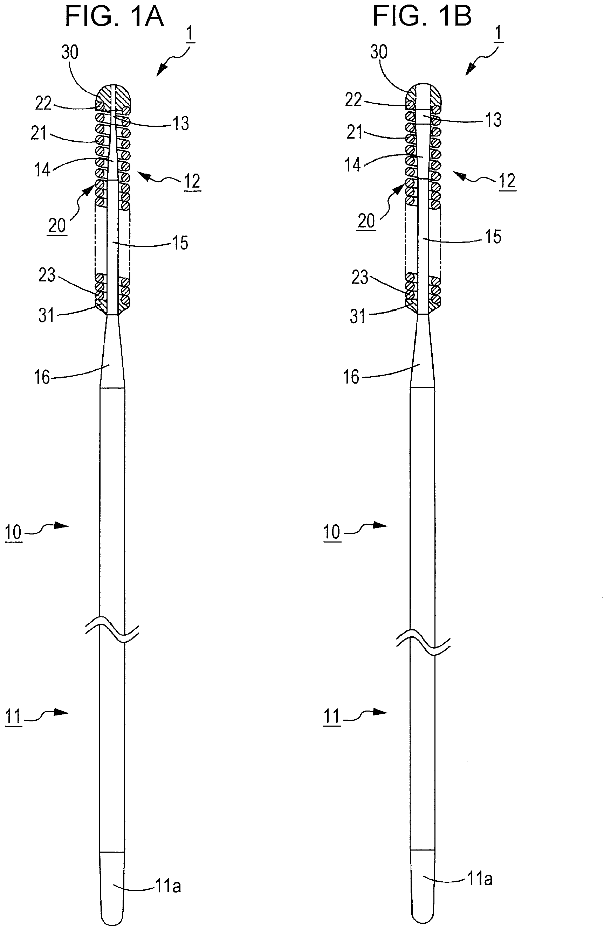

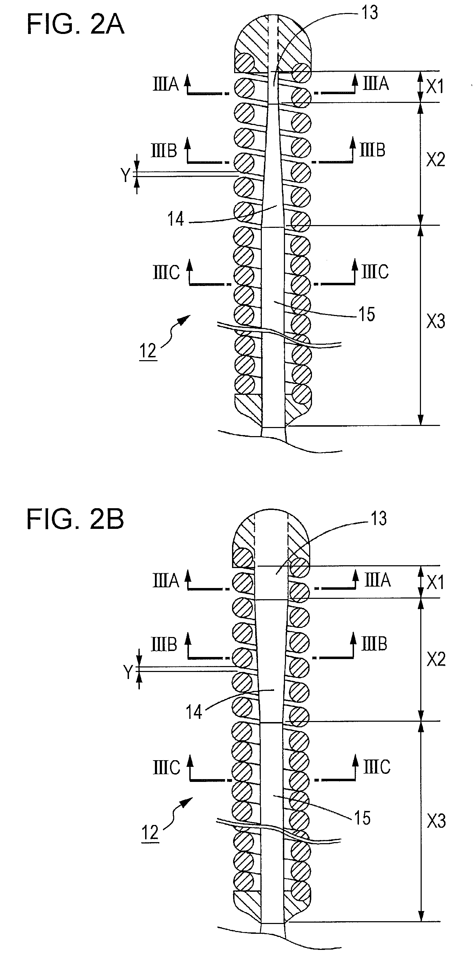

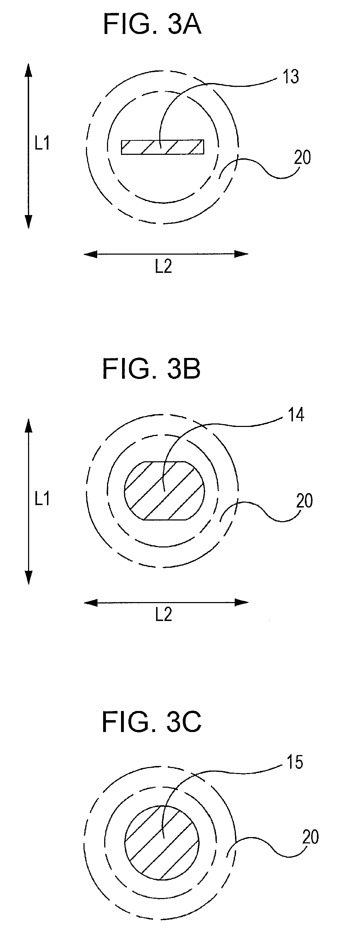

Fig. 1A is a schematic sectional view of a guidewire according to an embodiment of the present invention that is cut vertically along the longitudinal direction of the guidewire, and Fig. 1B is a schematic sectional view of the guidewire illustrated in Fig. 1A that is cut horizontally along the longitudinal direction, as viewed from another direction by rotating the guidewire by 90 degrees around the longitudinal axis of the guidewire. Fig. 2A is an enlarged view of a distal end portion of the guidewire illustrated in Fig. 1A, and Fig. 2B is an enlarged view of the distal end portion of the guidewire illustrated in Fig. 1B. Fig. 3A is a sectional view of a most distal end portion of the guidewire illustrated in Figs. 2A and 2B taken along line IIIA-IIIA, Fig. 3B is a sectional view of an intermediate portion of the guidewire illustrated in Figs. 2A and 2B taken along line IIIB-IIIB, and Fig. 3C is a sectional view of a cylindrical portion of the guidewire illustrated in Figs. 2A and 2B taken along IIIC-IIIC. In the following description, a distal portion of a guidewire and a distal end portion of a core shaft will be denoted by the same numeral, and a proximal portion of the guidewire and a proximal end portion of the core shaft will be denoted by the same numeral. A part or the entirety of a coil may be illustrated by broken line, instead of illustrating the entirety of the coil.

Referring to Figs. 1A and 1B, a guidewire 1 according to the present invention includes a core shaft 10 and a coil 20. The core shaft 10 has a diameter that decreases from a proximal end portion 11 toward a distal end portion 12. The coil 20 is wound around an outer periphery of the distal end portion 12.

Referring to Figs. 1A to 2B, the distal end portion 12 includes a most distal end portion 13 that is positioned at the most distal end and an intermediate portion 14 that is connected to the most distal end portion 13.

Referring to Fig. 3A, the cross-sectional shape of the most distal end portion 13 is a rectangular shape. Therefore, the most distal end portion 13 is easily bent when an external force is applied in vertical directions L1 in Fig. 3A, but is not easily bent when an external force is applied in horizontal directions L2. That is, the directionality of the flexibility of the most distal end portion 13 when an external force is applied is high, and the most distal end portion 13 has a high flexibility. In the present specification, the term "cross-sectional shape" refers to a cross-sectional shape of the core shaft when the core shaft is cut in a direction perpendicular to the longitudinal direction of the core shaft.

The cross-sectional shape of the intermediate portion 14, which is illustrated in Fig. 3B, gradually changes from the rectangular shape to a circular shape in a direction from the most distal end portion 13 toward the proximal end portion 11. Therefore, the intermediate portion 14 is likely to be bent more substantially uniformly than the most distal end portion 13 when an external force is applied either in the vertical directions L1 or in the horizontal directions L2 in Figs. 3B and 3C. That is, the directionality of the flexibility of the intermediate portion 14 is low.

The length X1 of the most distal end portion 13, which has a higher directionality of flexibility, is shorter than the length X2 of the intermediate portion 14, which has a lower directionality of flexibility. Therefore, the most distal end portion 13 not only has certain flexibility but also is less likely to be twisted when the proximal end portion 11 is rotated in either direction around the longitudinal axis of the core shaft 10. In the present specification, if a distal end brazed portion, which will be described below, is formed at the most distal end of the guidewire and a part of the most distal end portion is embedded in the distal end brazed portion, the length of the embedded part of most distal end portion is not included in "the length of the most distal end portion".

With the guidewire 1 including the core shaft 10 configured as described above, the directionality of the flexibility of the distal end portion 12 is low. Therefore, even when the proximal end portion 11 is continuously rotated, the distal end portion 12 is less likely to be twisted. Moreover, the distal end portion 12 has certain flexibility. Therefore, the guidewire 1 according to the present invention is not likely to damage an inner wall of a blood vessel and is not likely to make a hole in the inner wall.

### BRIEF DESCRIPTION OF THE DRAWINGS

Fig. 1A is a schematic sectional view of a guidewire according to an embodiment of the present invention that is cut vertically along the longitudinal direction of the guidewire, and Fig. 1B is a schematic sectional view of the guidewire illustrated in Fig. 1A that is cut horizontally along the longitudinal direction;
Fig. 2A is an enlarged view of a distal end portion of the guidewire illustrated in Fig. 1A, and Fig. 2B is an enlarged view of the distal end portion of the guidewire illustrated in Fig. 1B;
Fig. 3A is a sectional view of a most distal end portion of the guidewire illustrated in Figs. 2A and 2B taken along line IIIA-IIIA, Fig. 3B is a sectional view of an intermediate portion of the guidewire illustrated in Figs. 2A and 2B taken along line IIIB-IIIB, and Fig. 3C is a sectional view of a cylindrical portion of the guidewire illustrated in Figs. 2A and 2B taken along IIIC-IIIC;

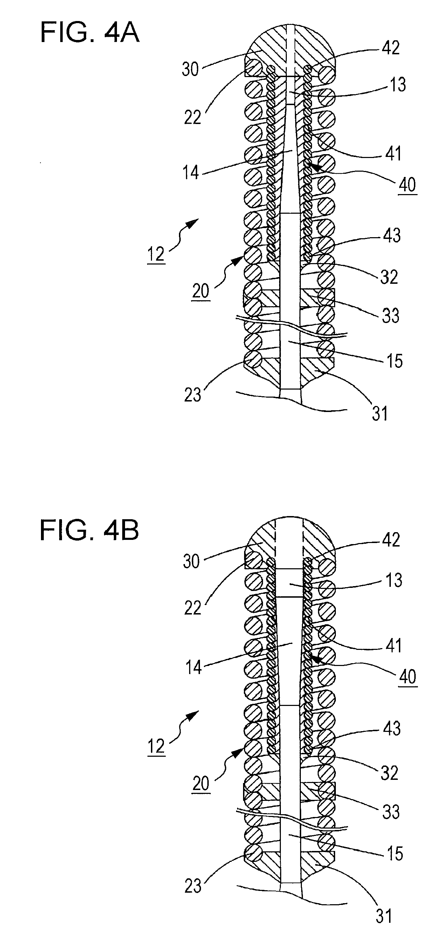

Fig. 4A is an enlarged view of a distal end portion of a guidewire according to another embodiment of the present invention, and Fig. 4B is an enlarged view of the distal end portion of the guidewire illustrated in Fig. 4A as viewed from another direction by rotating the guidewire by 90 degrees around the longitudinal axis of the guidewire; and
Fig. 5A is a schematic sectional view of a core shaft of an existing guidewire that is cut vertically along the longitudinal direction of the core shaft, Fig. 5B is a schematic sectional view of the core shaft illustrated Fig. 5A that is cut horizontally along the longitudinal direction, Fig. 5C is a sectional view of a most distal end portion of the core shaft illustrated in Figs. 5A and 5B taken along line VC-VC, and Fig. 5D is a sectional view of an intermediate portion of the core shaft illustrated in Figs. 5A and 5B taken along line VD-VD.

### DESCRIPTION OF THE PREFERRED EMBODIMENTS

### First Embodiment

Hereinafter, a guidewire according to a first embodiment of the present invention will be described with reference to the drawings. The guidewire according to the present embodiment has a structure similar to that of the guidewire according to the present invention described above, and the guidewire will be described with reference to Figs. 1A to 3C. Description the same as that of the guidewire according to the present invention may be omitted.

Referring to Figs. 1A to 3C, the guidewire 1 according to the present embodiment includes the core shaft 10 and the coil 20. The core shaft 10 has a diameter that decreases from the proximal end portion 11 toward the distal end portion 12. The coil 20 is wound around the outer periphery of the distal end portion 12. The distal end portion 12 includes the most distal end portion 13, which is positioned at the most distal end, and the intermediate portion 14, which is connected to the most distal end portion 13. The cross-sectional shape of the most distal end portion 13 is a rectangular shape, and the cross-sectional shape of the intermediate portion 14 gradually changes from the rectangular shape to a circular shape in a direction from the most distal end portion 13 toward the proximal end portion 11. The length of the most distal end portion 13 is shorter than the length of the intermediate portion 14. Hereinafter, the guidewire 1 according to the present embodiment will be described in detail.

### Structure of Core Shaft of Guidewire according to First Embodiment

Referring to Figs. 1A and 1B, the proximal end portion 11 has a cylindrical shape with a substantially uniform diameter. A connection portion 11a, to which an extension guidewire or the like can be connected, is formed at the most proximal end of the proximal end portion 11.

The most distal end of the proximal end portion 11 is connected to the most proximal end of the taper portion 16, and the most distal end of the taper portion 16 is connected to the most proximal end of the distal end portion 12. The taper portion 16 has a diameter that decreases from the proximal end portion 11 side toward the distal end portion 12 side. The largest diameter (the largest dimension in the cross-section) of the distal end portion 12 is shorter than the diameter of the proximal end portion 11.

Referring to Figs. 2A and 2B, the distal end portion 12 includes the most distal end portion 13 disposed at the most distal end, the intermediate portion 14 connected to the most distal end portion 13, and a cylindrical portion 15 connected to the intermediate portion 14.

Referring to Figs. 2A and 2B it is seen that the diameter of the cylindrical portion 15 remains constant over the length of the cylindrical portion 15, and that a radial or cross-sectional extension of the intermediate portion 14 decreases towards the most distal end portion 13 in the sectional view of Fig. 1A and increases towards the most distal end portion 13 in the sectional view of Fig. 1B.

As described above with reference to Fig. 3A, the cross-sectional shape of the most distal end portion 13 is a rectangular shape. To be specific, the shape of the most distal end portion 13 is a flat plate-like shape. Referring to Fig. 3A, the cross-sectional shape of the most distal end portion 13 cut at the middle thereof along the longitudinal direction is an elongated rectangular shape that is surrounded by two opposing long sides and two opposing short sides. Therefore, the directionality of the flexibility of the most distal end portion 13 is high, and the most distal end portion 13 is the most flexible part of the distal end portion 12. It is preferable that the length X1 of the most distal end portion 13 be equal to or longer than 1 mm and shorter than 3 mm.

As described above with reference to Fig. 3B, the cross-sectional shape of the intermediate portion 14 gradually changes from the rectangular shape to a circular shape in a direction from the most distal end portion 13 toward the proximal end portion 11. That is, the cross-sectional shape of a part of the intermediate portion 14 nearer to the most distal end portion 13 is substantially rectangular, and the cross-sectional shape of a part of the intermediate portion 14 nearer to the cylindrical portion 15 is substantially circular. Referring to Fig. 3B, the cross-sectional shape of the intermediate portion 14 cut at the middle thereof along the longitudinal direction is a deformed rectangular shape surrounded by two opposing sides and two opposing arcs. Therefore, the directionality of the flexibility of the intermediate portion 14 is low, and the flexibility of the intermediate portion 14 is lower than that of the most distal end portion 13. It is preferable that the length X2 of the intermediate portion 14 be equal to or longer than 3 mm and shorter than 4 mm.

The length X1 of the most distal end portion 13 is shorter than the length X2 of the intermediate portion 14.

The cross-sectional shape of the cylindrical portion 15 is a circular shape. Referring to Fig. 3C, the cross-sectional shape of the cylindrical portion 15 cut at any position along the longitudinal direction is, for example, a circular shape. Therefore, the directionality of the flexibility of the cylindrical portion 15 is considerably low, and the flexibility of the cylindrical portion is lower than those of the most distal end portion 13 and the intermediate portion 14. It is preferable that the length X3 of the cylindrical portion 15 be equal to or longer than 40 mm and shorter than 60 mm.

### Structure of Coil of Guidewire according to First Embodiment

Referring to Figs. 1A and 1B, the coil 20 is formed by helically winding a single or a plurality of strands 21. The coil 20 is a tube-like member having a through-hole extending therethrough.

The distal end portion 12 is inserted through the coil 20, and the coil 20 covers the distal end portion 12. The distal end portion 12 and the inner wall of the coil 20 are disposed so as to be separated from each other with a predetermined distance therebetween.

Referring to Figs. 2A and 2B, in a section of the coil 20 that is wound around outer peripheries of the most distal end portion 13 and the intermediate portion 14 the pitch Y at which the strand 21 is wound is substantially constant and so that adjacent turns of the strand 21 are spaced apart from each other.

In a section of the coil 20 that is wound around the outer periphery of the cylindrical portion 15, the coil 20 is densely wound so that adjacent turns of the strand 21 are in contact with each other.

A distal end 22 of the coil 20 and the most distal end portion 13 of the core shaft 10 are fixed to each other through a distal end brazed portion 30 having a semispherical shape. A proximal end 23 of the coil 20 and a proximal end of the cylindrical portion 15 of the core shaft 10 are fixed to each other through a proximal end brazed portion 31. A single or a plurality of intermediate brazed portions may be formed between the distal end brazed portion 30 and the proximal end brazed portion 31.

### Method of Manufacturing Guidewire according to First Embodiment

The guidewire according to the present embodiment can be manufactured by, for example, making a core shaft by taper-cutting or pressing a wire so as to form the shape described above, inserting a distal end portion of the core shaft into a coil, and brazing the core shaft and the coil to each other at predetermined positions.

Advantages of the guidewire according to the present embodiment are as follows.
(1) In the guidewire according to the present embodiment, the length of the most distal end portion of the core shaft, which has a high flexibility and is more likely to become twisted, is shorter than the length of the intermediate portion, which has a low flexibility and is less likely to become twisted. Therefore, the distal end portion of the core shaft has a certain degree of flexibility but is less likely to become twisted. Due to such a structure of the core shaft, even when the proximal end portion of the guidewire according to the present embodiment is continuously rotated, the distal portion of the guidewire can easily follow the rotation, so that the distal portion is less likely to become twisted. Therefore, the guidewire according to the present embodiment can be prevented from springing forward, and the guidewire is not likely to damage an inner wall of a blood vessel and is not likely to make a hole in the inner wall.
(2) It is preferable that the length of the most distal end portion be equal to or longer than 1 mm and shorter than 3 mm and the length of the intermediate portion be equal to or longer than 3 mm and shorter than 4 mm, because, in this case, the advantage described in (1) can be particularly obtained. In contrast, if the length of the most distal end portion is shorter than 1 mm, the most distal end portion is too short and the flexibility may decrease. If the length of the most distal end portion is longer than 3 mm, the most distal end portion is too long and springing forward may occur. If the length of the intermediate portion is shorter than 3 mm, the intermediate portion is too short and the shape of the core shaft changes sharply from the most distal end portion to the cylindrical portion, and therefore the vicinity of the intermediate portion may become damaged due to a bending force or a twisting force. In contrast, if the length of the intermediate portion is longer than 4 mm, the intermediate portion is too long, and a torque generated when the proximal end of the guidewire is rotated may not be efficiently transferred to the most distal end of the distal portion.
(3) In the coil, in a section of the coil that is wound around the outer peripheries of the most distal end portion and the intermediate portion the pitch at which the strand is wound is substantially constant and so that adjacent turns of the strand of the coil are spaced apart from each other, so that this section of the coil has a higher flexibility. In addition, as described above, the most distal end portion and the intermediate portion of the core shaft have flexibilities that are higher than that of the cylindrical portion. Therefore, the distal portion (in particular, the most distal end of the distal portion) of the guidewire has a higher flexibility.
(4) In a section of the coil that is wound around the outer periphery of the cylindrical portion, the coil is densely wound so that adjacent turns of the strand are in contact with each other. Therefore, this section of the coil is not easily twisted even when a twisting force is applied to the section, and a torque generated when the proximal portion of the guidewire is rotated can be efficiently transferred to the most distal end of the distal portion.

### Second Embodiment

Hereinafter, a guidewire according to a second embodiment of the present invention will be described with reference to the drawings. The guidewire according to the present embodiment further includes a stranded wire tube, and a core shaft that extends through the stranded wire tube. The stranded wire tube and the core shaft are fixed to each other through a first fixing portion. The coil and the core shaft are fixed to each other through a second fixing portion that is disposed so as to be separated from the first fixing portion. In other respects, the guidewire according to the second embodiment has a structure similar to that of the guidewire according to the first embodiment. Therefore, description of the structure the same as that of the first embodiment will be omitted.

Fig. 4A is an enlarged view of a distal end portion of a guidewire according to the second embodiment of the present invention, and Fig. 4B is an enlarged view of the distal end portion of the guidewire illustrated in Fig. 4A as viewed from another direction by rotating the guidewire by 90 degrees around the longitudinal axis of the guidewire.

Referring to Fig. 4A and 4B, the guidewire according to the present embodiment further includes a stranded wire tube 40.

The stranded wire tube 40, which is formed by stranding a plurality of strands 41, is a tubular body having a through-hole therein. Therefore, the stranded wire tube 40 is flexible and is less likely to become plastically deformed. Moreover, when one end of the stranded wire tube 40 is rotated, the other end of the stranded wire tube 40 is easily rotated so as to follow the rotation, and the stranded wire tube 40 has a torque transmission ability higher than that of the coil 20.

The stranded wire tube 40 is disposed in the coil 20. The distal end portion 12 of the core shaft (including the most distal end portion 13, the intermediate portion 14, and the cylindrical portion 15) extends through the stranded wire tube 40.

The distal end 22 of the coil 20, the most distal end portion 13 of the core shaft, and a distal end 42 of the stranded wire tube 40 are fixed to one another through the distal end brazed portion 30 having a semispherical shape. The proximal end 23 of the coil 20 and the proximal end of the cylindrical portion 15 of the core shaft 10 are fixed to each other through the proximal end brazed portion 31. A proximal end 43 of the stranded wire tube 40 and the cylindrical portion 15 of the core shaft are fixed to each other through a first fixing portion 32. An intermediate portion of the coil 20 and the cylindrical portion 15 of the core shaft are fixed to each other through a second fixing portion 33 that is disposed so as to be separated from the first fixing portion 32 toward the proximal end portion by a predetermined distance. In regions in which the first fixing portion 32 and the second fixing portion 33 are formed, the distal end portion 12 and the coil 20 are slightly less likely to be uniformly bent. As with the distal end brazed portion 30 and the proximal end brazed portion 31, the first fixing portion 32 and the second fixing portion 33 may be formed as brazed portions. Method of Manufacturing Guidewire according to Second Embodiment

The guidewire according to the present embodiment can be manufactured, for example, as follows. First, a core shaft made by a method similar to that of making the first embodiment is prepared, the distal end portion of the core shaft is inserted into the stranded wire tube, and the distal end portion and the stranded wire tube are brazed to each other at predetermined positions. Then, the distal end portion is inserted into the coil so that the stranded wire tube is covered by the coil, and the core shaft and the coil are brazed to each other at predetermined positions.

Advantages of the guidewire according to the present embodiment are as follows. With the guidewire according to the present embodiment, the advantages the same as those of the first embodiment, which are described in (1) to (4), are obtained. In addition, the guidewire has the following advantages (5) and (6).
(5) Although the distal portion of the guidewire has a high flexibility, the distal portion is less likely to become plastically deformed, because the core shaft extends through the stranded wire tube that is flexible and is less likely to become plastically deformed. The guidewire has a high torque transmission ability, because the stranded wire tube having a high torque transmission ability is used.
(6) The stranded wire tube and the core shaft are fixed to each other through the first fixing portion, and the coil and the core shaft are fixed to each other through the second fixing portion, which is disposed so as to be separated from the first fixing portion. Thus, the first fixing portion and the second fixing portion, which are slightly less likely to be uniformly bent, are formed so as to be separated from each other. Therefore, as compared to the case where the first fixing portion and the second fixing portion are formed so as to overlap each other, the distal portion of the guidewire is more likely to be uniformly bent, and therefore the guidewire has a high operability.

### Other Embodiments

In the guidewire according to the present invention, the cross-sectional shape of the most distal end portion is a rectangular shape. However, the cross-sectional shape of the most distal end portion of the core shaft is not limited to an elongated rectangular shape illustrated in Fig. 3A, and may be any shape as long as the most distal end portion has a directionality in the flexibility. For example, the cross-sectional shape may be an elongated deformed rectangle surrounded by two opposing long sides and two opposing short arcs, or the like.

In the guidewire according to the present invention, it is preferable that the pitch at which the strand is wound in a section of the coil that is wound around the outer peripheries of the most distal end portion and the intermediate portion be substantially constant. However, the coil may be densely wound in this section of the coil so that adjacent turns of the strand are in contact with each other.

If the guidewire according to the present invention includes the stranded wire tube, the stranded wire tube may cover the most distal end portion, the intermediate portion, and the cylindrical portion as described above. However, the stranded wire tube may cover only the most distal end portion, or may cover only the most distal end portion and the intermediate portion.

The strand of the stranded wire tube may be made from, for example, a stainless steel, a superelastic alloy such as a Ni-Ti alloy, a piano wire, or a tungsten wire. Examples of the stainless steel include a martensitic stainless steel, a ferritic stainless steel, an austenitic stainless steel, an austenitic-ferritic duplex stainless steel, and a precipitation hardening stainless steel. Among these, an austenitic stainless steel is preferable, and in particular, SUS304, SUS316, or SUS316L is more preferable.

In the guidewire according to the present invention, the core shaft may be made from, for example, a stainless steel, a superelastic alloy such as a Ni-Ti alloy, a piano wire, or a tungsten wire. Examples of the stainless steel include a martensitic stainless steel, a ferritic stainless steel, an austenitic stainless steel, an austenitic-ferritic duplex stainless steel, and a precipitation hardening stainless steel. Among these, an austenitic stainless steel is preferable, and in particular, SUS304, SUS316, or SUS316L is more preferable.

In the guidewire according to the present invention, it is preferable that the strand of the coil be made from a stainless steel such as a martensitic stainless steel, a ferritic stainless steel, an austenitic stainless steel, an austenitic-ferritic duplex stainless steel, and a precipitation hardening stainless steel; a superelastic alloy such as a Ni-Ti alloy; or platinum, gold, tungsten, or the like, which is a radiopaque metal.

In the guidewire according to the present invention, the coil may have a tapering shape such that the diameter of the coil decreases from the proximal end toward the distal end. A guidewire including such a coil is preferable because the guidewire can be inserted into a hard lesion such as chronic total occlusion.

In the guidewire according to the present invention, it is preferable that the brazed portions be made from, for example, an aluminium alloy, silver, gold, zinc, a Sn-Pb alloy, a Sn-Au alloy, a Pb-Ag alloy, a Sn-Ag alloy, or the like. Among these, gold, a Sn-Au alloy, and a Sn-Ag alloy are particularly preferable. This is because the strengths of the brazed portions are increased by using such a material.

In the guidewire according to the present invention, the outer surface of the guidewire may be coated with a hydrophilic material. This is because, with such a coating, friction between the guidewire and the inner wall of a guiding catheter, a lumen, or a body tissue can be reduced and the guidewire can be smoothly moved.

Examples of the hydrophilic material include, a cellulose polymer, a polyethylene oxide polymer, a maleic anhydride polymer (for example, a maleic anhydride copolymer such as a methyl vinyl ether/maleic anhydride copolymer), an acrylamide polymer (for example, a polyacrylamide, a polyglycidyl methacrylate/dimethylacrylamide blockcopolymer), a water-soluble nylon, polyvinyl alcohol, polyvinylpyrrolidone, and a hyaluronate. Among these, a hyaluronate is more preferable.

## Claims

1. A guidewire (1) comprising:
a core shaft (10) having a diameter that decreases from a proximal end portion (11) toward a distal end portion (12); **characterized by**
a coil (20) wound around an outer periphery of the distal end portion (12),
wherein the distal end portion (12) comprises
a most distal end portion (13) that is positioned at a most distal end of the distal end portion (12), and
an intermediate portion (14) that is connected to the most distal end portion (13),
wherein a cross-sectional shape of the most distal end portion (13) is a rectangular shape,
wherein a cross-sectional shape of the intermediate portion (14) gradually changes from the rectangular shape to a circular shape in a direction from the most distal end portion (13) toward the proximal end portion (11), and
wherein a length (X1) of the most distal end portion (13) is shorter than a length (X2) of the intermediate portion (14).

2. The guidewire (1) according to Claim 1,
wherein the length (X1) of the most distal end portion (13) is equal to or longer than 1 mm and shorter than 3 mm, and the length (X2) of the intermediate portion (14) is equal to or longer than 3 mm and shorter than 4 mm.

3. The guidewire (1) according to Claim 1 or 2,
wherein in a section of the coil (20) that is wound around outer peripheries of the most distal end portion (13) and the intermediate portion (14) a pitch (Y) at which a strand (21) is wound is constant.

4. The guidewire (1) according to any one of Claims 1 to 3, further comprising:
a stranded wire tube (40) disposed inside the coil (20),
wherein the core shaft (10) extends through the stranded wire tube (40).

5. The guidewire (1) according to Claim 4,
wherein the stranded wire tube (40) and the core shaft (10) are fixed to each other through a first fixing portion (32), and
wherein the coil (20) and the core shaft (10) are fixed to each other through a second fixing portion (33) that is disposed so as to be separated from the first fixing portion (32).
